# EUROPÄISCHE PATENTANMELDUNG

(11) **EP 2 189 170 A1**
(43) Veröffentlichungstag der Anmeldung: **26.05.2010**
(21) Anmeldenummer: 09174334.4
(22) Anmeldetag: 28.10.2009
(51) Int. Cl.: A61L 31/08, C25D 11/30

(54) **Verfahren zur Herstellung einer korrosionshemmenden Beschichtung auf einem Implantat aus einer biokorrodierbaren Magnesiumlegierung sowie nach dem Verfahren hergestelltes Implantat**

(30) Priorität: 21.11.2008 DE 102008043970
(71) Anmelder: Biotronik VI Patent AG, 6341 Baar (CH)
(72) Erfinder: Kappelt, Gerhard, 91054, Erlangen (DE); Kurze, Peter, 52385, Nideggen (DE); Banerjee, Dora, 50171, Kerpen (DE); Adden, Nina, 90427, Nürnberg (DE)
(74) Vertreter: Lindner-Vogt, Karin L.

(57) **Zusammenfassung**

Die Erfindung betrifft Verfahren zur Herstellung korrosionshemmender Beschichtungen auf einem Implantat aus einer biokorrodierbaren Magnesiumlegierung sowie nach dem Verfahren erhaltene oder erhältliche Implantate.

## Beschreibung

Die Erfindung betrifft zwei Verfahren zur Herstellung korrosionshemmender Beschichtungen auf einem Implantat aus einer biokorrodierbaren Magnesiumlegierung sowie nach dem Verfahren erhaltene oder erhältliche Implantate.

### Technologischer Hintergrund und Stand der Technik

Implantate finden in vielfältiger Ausführungsform in der modernen Medizintechnik Anwendung. Sie dienen unter anderem der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

Die Implantation von Stents ist eine der wirkungsvollsten therapeutischen Maßnahmen bei der Behandlung von Gefäßerkrankungen. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper jedes Implantats, insbesondere von Stents, besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden. Für die Zwecke der vorliegenden Erfindung sind lediglich degradierbare/resorbierbare, metallische Implantatwerkstoffe von Interesse, die nachfolgend als biokorrodierbare metallische Werkstoffe bezeichnet werden.

Der Einsatz biokorrodierbarer metallischer Werkstoffe bietet sich insbesondere schon deshalb an, da häufig nur ein zeitweiliger Verbleib des Implantats im Körper zur Erfüllung des medizinischen Zweckes erforderlich ist. Implantate aus permanenten Werkstoffen, also Werkstoffen, die im Körper nicht abgebaut werden, sind gegebenenfalls wieder zu entfernen, da es mittel- und langfristig auch bei hoher Biokompatibilität zu Abstoßungsreaktionen des Körpers kommen kann.

Ein Ansatz zur Vermeidung eines weiteren chirurgischen Eingriffs besteht demnach darin, das Implantat ganz oder in Teilen aus einem biokorrodierbaren metallischen Werkstoff zu formen. Unter Biokorrosion werden Vorgänge verstanden, die durch die Anwesenheit von Körpermedien bedingt sind und die zu einem allmählichen Abbau der aus dem Werkstoff bestehenden Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Die Abbauprodukte haben, wie beispielsweise beim Magnesium, im günstigsten Fall sogar eine positive therapeutische Wirkung auf das umgebende Gewebe. Geringe Mengen nicht resorbierbarer Legierungsbestandteile sind - sofern sie nicht toxisch sind - tolerierbar.

Bekannte biokorrodierbare metallische Werkstoffe umfassen Reineisen und biokorrodierbare Legierungen der Hauptelemente Magnesium, Eisen, Zink, Molybdän und Wolfram. In DE 197 31 021 A1 wird unter anderem vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Für die Zwecke der vorliegenden Erfindung sind lediglich biokorrodierbare Magnesiumlegierungen von Interesse.

EP 1 419 793 B1 beschreibt den Einsatz einer biokorrodierbaren Magnesiumlegierung mit einem Anteil von Magnesium >90 Gew.%, Yttrium 3.7 - 5.5 Gew.%, Seltenerdmetallen 1.5 - 4.4 Gew.% und Rest < 1 Gew.% zur Herstellung eines Stents.

EP 1 842 507 A1 beschreibt ein Implantat aus einem Grundkörper, der aus einer yttriumfreien und gadoliniumhaltigen Magnesiumlegierung besteht. Die Legierung kann ferner Neodym (Nd), Zink (Zn), Zirkonium (Zr) und Calcium (Ca) enthalten. Die Legierung enthält vorzugsweise 1.0 bis 5.0 Gew.% Gd und 1.0 bis 5.0 Gew.% Nd, um die Cytotoxizität auf geringem Niveau zu belassen und mechanische Eigenschaften wie Festigkeit, Härte und Duktilität, sowie die Prozessierbarkeit des Werkstoffs zu verbessern. Ein Gehalt von Zn als auch Zr beträgt vorzugsweise 0.1 bis 3.0 Gew.%, um eine homogene Verteilung der Elemente in der Legierung sicherzustellen.

Biologisch abbaubare Gefäßstützen (Stents) aus Magnesiumlegierungen sind bereits in klinischen Studien erprobt worden. So wurde eine Magnesiumlegierung, die Yttrium und Seltene Erden enthält, technische Bezeichnung WE43, verwendet. Bei der Verwendung dieser Legierung, die in Tierversuchen auch bereits in anderen Bereichen der Implantologie erprobt wurde, bereiten aber einige Eigenschaften in einer physiologischen Umgebung nach wie vor Probleme; diese WE-Legierungen zeigen speziell eine zu schnelle Degradation in physiologischen Medien. Die relativ rasche Biokorrosion der Magnesiumlegierungen, insbesondere im Bereich mechanisch stark belasteter Strukturen, limitiert den Einsatz der Implantate.

Sowohl die Grundlagen der Magnesiumkorrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierung einen leicht erhöhten Korrosionswiderstand in Meerwasser verleiht.

Ein anderer Ansatzpunkt sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden unter dem Gesichtpunkt eines technischen Einsatzes des Formkörpers - jedoch nicht medizintechnischen Einsatzes in biokorrodierbaren Implantaten in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, Ionenimplantation oder Lackieren.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Inhibierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung einer Korrosionsschutzschicht nicht. Ferner müssen für einen medizintechnischen Einsatz auch toxikologische Aspekte berücksichtigt werden. Des Weiteren sind korrosive Prozesse stark von dem Medium abhängig, in dem sie ablaufen, und daher ist eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich. Schließlich dürften bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Nahtmaterial oder Clips im Gebrauch mechanisch verformt, so dass der Teilprozess der Spannungsriss-Korrosion beim Abbau dieser Formkörper erhebliche Bedeutung haben sollte.

DE 101 63 106 A1 sieht vor, den Magnesiumwerkstoff durch Modifikation mit Halogeniden in seiner Korrosivität zu verändern. Der Magnesiumwerkstoff soll zur Herstellung medizinischer Implantate verwendet werden. Bevorzugt ist als Halogenid ein Fluorid. Die Modifikation des Werkstoffs wird durch Zulegieren salzförmiger Halogenverbindungen erreicht. Es wird demnach die Zusammensetzung der Magnesiumlegierung durch Zugabe der Halogenide verändert, um die Korrosionsrate zu mindern. Dementsprechend wird der gesamte, aus einer solchen modifizierten Legierung bestehende Formkörper ein verändertes Korrosionsverhalten besitzen. Durch das Zulegieren können zudem weitere Werkstoffeigenschaften beeinflusst werden, die bei der Verarbeitung von Bedeutung sind oder die mechanischen Eigenschaften des aus dem Werkstoff entstehenden Formkörpers prägen.

Der vorliegenden Erfindung liegt die Aufgabe zugrunde, zumindest alternative oder vorzugsweise verbesserte Verfahren zur Herstellung einer korrosionshemmenden Beschichtung auf einem Implantat aus einer biokorrodierbaren Magnesiumlegierung bereitzustellen. Die durch das Verfahren erschaffene korrosionshemmende Beschichtung soll nur eine temporäre Inhibition, aber nicht vollständige Unterbindung der Korrosion des Werkstoffs in physiologischer Umgebung bewirken. Insbesondere soll ein hinsichtlich seines Korrosionsverhaltens verbessertes Implantat aus einer biokorrodierbaren Magnesiumlegierung bereitgestellt werden.

### Zusammenfassung der Erfindung

Das erfindungsgemäße Implantat löst oder mindert ein oder mehrere der zuvor geschilderten Aufgaben. Die Erfindung geht von einem Implantat aus, das ganz oder in Teilen aus einer biokorrodierbaren Magnesiumlegierung besteht.

Diese Aufgabe wird gemäß einer ersten Variante durch das erfindungsgemäße Verfahren zur Herstellung einer korrosionshemmenden Beschichtung auf einem Implantat aus einer biokorrodierbaren Magnesiumlegierung gelöst. Das erste Verfahren umfasst die Schritte:
(i) Bereitstellen des Implantats aus einer biokorrodierbaren Magnesiumlegierung; und
(ii) Anodische plasmachemische Behandlung der Implantatsoberfläche in einem wässrigen, fluoridfreien Elektrolyten, der zumindest Ammoniak (NH₃), Phosphorsäure (H₃PO₄) und Borsäure (H₃BO₃) enthält.

Ein hierzu alternatives, zweites Verfahren zur Herstellung einer korrosionshemmenden Beschichtung auf einem Implantat aus einer biokorrodierbaren Magnesiumlegierung umfasst die Schritte:
(i) Bereitstellen des Implantats aus einer biokorrodierbaren Magnesiumlegierung; und
(ii) Anodische Behandlung der Implantatsoberfläche mit einem wässrigen Elektrolyten, der zumindest Natriumpermanganat (NaMnO₄) und Ammoniumvanadat (NH₄VO₃) enthält.

Es hat sich gezeigt, dass die Erzeugung einer Beschichtung in den genannten Verfahrensweisen nicht zur Ausbildung einer vollständig oder weitgehend die Korrosion in physiologischer Umgebung inhibierenden Schutzschicht führt. Mit anderen Worten, in physiologischer Umgebung erfolgt dennoch eine Korrosion des Implantats, jedoch mit deutlich verzögerter Geschwindigkeit. Die korrosionshemmende Beschichtung entsteht jeweils durch oberflächennahe Konversion des Werksstoffs des Implantats; es erfolgt also kein Auftrag von Material auf eine Oberfläche des Implantats, sondern es findet eine chemische Umwandlung (Konversion) der metallischen Oberfläche und der verschiedenen Bestandteile der Elektrolyten statt.

Ein weiterer bedeutsamer und gemeinsamer Vorteil der vorgenannten Verfahrensführung liegt darin, dass die entstehende Passivierungsschicht an der Außenseite porös ist. Damit ist die Einbettung pharmazeutisch aktiver Substanzen in Reinform oder entsprechender Galenik möglich, was gerade mit Hinsicht auf die Verwendung in Implantaten, insbesondere Stents, von besonderer Bedeutung ist.

Schließlich ist vorteilhaft, dass die erzeugten Passivierungsschichten eine hohe Haftung zeigen, aber nicht spröde sind und bei mechanischer Beanspruchung zerplatzen. Es konnte vielmehr bei Vorstudien an Stents gezeigt werden, dass auch erhebliche mechanische Belastungen toleriert werden, ohne dass die Passivierungsschicht eine signifikante Verschlechterung erfährt oder Teile der Passivierungsschicht fragmentieren.

Die erste Verfahrensvariante über eine anodische plasmachemische Behandlung der Implantatoberfläche lehnt sich in modifizierter Weise an ein an sich bekanntes technisches Passivierungsverfahren an, dass in der Literatur auch als anodische Oxidation unter Funkenentladung (ANOF), spark discharges in electrolytes, anodic spark deposition (ASD), Micro-arc oxidation, High-voltage anodizing, plasma-electrolytic oxidation (PEO) und plasmachemische Anodisation bezeichnet wird. Bei der anodisch plasmachemischen Oberflächenveredelung werden Magnesiumlegierungen mit einer als Verschleiß- und Korrosionsschutz dienenden Oxidkeramikschicht veredelt. Die Oberflächenveredelung ist ein elektrolytisches Verfahren, bei der das Werkstück als Anode geschaltet wird und die Oberfläche des Werkstücks in entsprechende Oxide umgewandelt wird. Als Elektrolyte werden Salzlösungen verwendet. Die Anodisation erfolgt über Plasmaentladungen im Elektrolyten an der Oberfläche des zu beschichtenden Teiles. Durch Einwirkung des im Elektrolyten erzeugen Sauerstoffplasmas auf die Metalloberfläche wird das Metall partiell in kurzer Zeit erschmolzen und es entsteht ein fest haftender Oxidkeramik-Metallverbund auf dem Werkstück.

Strukturell besteht die entstehende Passivierungsschicht bei anodisch plasmachemischer Behandlung aus einer ersten dünnen Schicht, der sogenannten Sperrschicht, die in direktem Kontakt zum Metallsubstrat steht. Auf dieser Sperrschicht befindet sich eine porenarme Oxidkeramikschicht, auf der wiederum eine etwa gleich dicke porenreiche Oxidkeramikschicht ausgebildet wird. Letztere eignet sich insbesondere zur Imprägnierung mit therapeutisch aktiven Substanzen in Reinform oder entsprechender Galenik.

Eine Schlüsselfunktion bei der anodisch plasmachemischen Behandlung hat der eingesetzte Elektrolyt. Zum einen müssen die Ingredienzien des Elektrolyten so abgestimmt werden, dass es überhaupt zu einer Zündung im Sinne des Verfahrens kommt. Und zum anderen sind die dann startenden Prozesse mit Hinsicht auf die erfindungsgemäße Zweckbestimmung auszulegen. Es wurde nun überraschenderweise gefunden, dass die Gegenwart von HF, das in allen herkömmlichen Verfahren essentieller Bestandteil ist, zu einer für die speziellen Zwecke der Oberflächenveredelung von biokorrodierbaren Implantaten aus Magnesiumlegierungen ungeeignet stark aufgerauten und ungleichmäßigen Oberflächenstruktur führt. Dies hat zur Folge, dass gerade bei mechanisch beanspruchten Implantaten eine frühzeitige und unerwünschte Fragmentierung zu beobachten ist. Es konnte nun überraschenderweise festgestellt werden, dass der Verzicht auf HF, also der Einsatz eines fluoridfreien Elektrolyts, zu sehr gleichmäßigen Passivierungsschichten führt, wenn der Elektrolyt gleichzeitig Ammoniak, Phosphorsäure und Borsäure enthält.

Ein weiterer, wichtiger Verfahrensparameter der anodisch plasmachemischen Behandlung ist die angelegte Spannung während des Prozesses. So hat es sich vorliegend als vorteilhaft erwiesen, wenn die maximale Endspannung im Bereich von 100 bis 400 Volt liegt, wobei unter Gleichstrom gearbeitet wird. Die Stromdichten liegen vorzugsweise bei 1 bis 5 A/dm², so dass Schichtbildungsgeschwindigkeiten von ca. 1 µm/min erreicht sind. Weiterhin liegt die Elektrolyttemperatur vorzugsweise im Bereich von 30°C bis 50°C.

Unter den genannten Bedingungen ließen sich wesentlich feinere Funken beobachten als dies in konventionellen Applikationen des anodisch plasmachemischen Verfahrens der Fall ist. Dies mag auch eine Erklärung für den homogeneren Schichtaufbau sein. Durch die Abwesenheit des Fluorids ist die Schicht weniger spröde, da kein MgF₂ gebildet werden kann. Auch auf eine sonst übliche Vorbehandlung des Werkstoffes mit Flusssäure zur Aktivierung wurde verzichtet, was überraschenderweise keinen negativen Einfluss auf das Verfahren hatte.

Auch die zweite Verfahrensvariante der anodischen Behandlung mit einem wässrigen Elektrolyten, der zumindest Natriumpermanganat und Ammoniumvanadat enthält, führte zu vergleichbaren Strukturen. Die Schichtdicke der sich bildenden Passivierungsschicht betrug ca. 2 bis 3 µm und erwies sich als haftfest, jedoch nicht spröde, so dass ein Abplatzen bei mechanischer Beanspruchung nicht zu beobachten war.

Vorzugsweise liegt ein Anteil von Natriumpermanganat im Elektrolyten im Bereich von 1 bis 5 g/l. Ein Anteil von Ammoniumvanadat beträgt vorzugsweise 0.5 bis 3 g/l. Die Behandlungstemperatur liegt vorzugsweise im Bereich von 20°C bis 40°C. Es wird vorzugsweise eine Gleichspannung im Bereich von 1 bis 15 V angelegt.

Optional enthalten die Elektrolytslösungen Puffer, insbesondere alkalische Puffer wie ED-TA, Ethylendiamin, Hexamethylentetramin. Alkalische Puffer unterstützen die Ausbildung einer Barriereschicht durch ihr hohes Angebot an OH⁻-Ionen. Weiterhin wirken sie sich günstig auf die Stabilität der Elektrolyten aus.

Das Implantat besteht ganz oder in zumindest zu Teilen aus der biokorrodierbare Magnesiumlegierung. Unter Legierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Die Magnesiumlegierung ist so in ihrer Zusammensetzung zu wählen, dass sie biokorrodierbar ist. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6.8 g/l, CaCl₂ 0.2 g/l, KCl 0.4 g/l, MgSO₄ 0.1 g/l, NaHCO₃ 2.2 g/l, Na₂HPO₄ 0.126 g/l, NaH₂PO₄ 0.026 g/I). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und mechanischer Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden eine einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt in besonderem Maße für Stents, welche zum Zeitpunkt der Implantation lokal hohen plastischen Verformungen ausgesetzt sind. Konventionelle Ansätze mit starren korrosionshemmenden Schichten sind für derartige Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Fall von Koronarstents sollte die mechanische Integrität der Struktur über ein Zeitraum von drei Monaten nach Implantation aufrechterhalten werden.

Ein weiterer Aspekt der Erfindung bezieht sich auf Implantate, die über die zuvor beschriebenen Verfahren erhalten oder erhältlich sind.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats (Dilatation) hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtungen ganz oder zumindest weitgehend diesen Anforderungen genügt.

Vorzugsweise weist die durch Behandlung mit der Konversionslösung erhältliche korrosionshemmende Beschichtung eine Schichtdicke in Bereich von 1 µm bis 10 µm auf.

Die Erfindung wird nachfolgend anhand von Ausführungsbeispielen und den dazugehörigen Illustrationen näher erläutert. Es zeigen:
- Figur 1: Querschliff durch eine Stentstrebe bei anodisch plasmachemischer Behandlung in konventioneller Weise und
- Figur 2: Querschliff durch eine Stentstrebe bei erfindungsgemäßer anodisch plasmachemischer Behandlung.

### Ausführungsbeispiel 1 - Anodische plasmachemische Oxidation

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Isopropanol unter Ultraschall gewaschen. Nach mehrfachen Spülen mit deionisiertem Wasser wurde der Stent als Anode gepolt und in eine wässrige Elektrolytlösung der folgenden Zusammensetzung getaucht:
60 g/l H₃PO₄
35 g/l H₃BO₃
72.8 g/l NH₃
350 g/l Hexamethylentetramin

Hexamethylentetramin diente der Stabilisierung eines Sauerstofffilms an der Anode und als pH-Puffer.

Die Parameter der anodischen Oxidation waren:

| | |
|---|---|
| Stromdichte | 1.4 A/dm² |
| Badtemperatur | 40°C |
| Max. Spannung | 340 V |

Nach 10 Minuten Expositionszeit wurde der Stent mit deionisiertem Wasser gespült und danach getrocknet. Die Beschichtung auf den Stents wies eine Schichtdicke von ca. 5 bis 8 µm auf.

### Vergleichsbeispiel - Anodische plasmachemische Oxidation in Gegenwart von HF

Zum Vergleich wurden Stents unter denselben Bedingungen, wie unter Ausführungsbeispiel 1 aufgeführt, behandelt, jedoch mit dem Unterschied, dass der Elektrolyt 30 g/l Flusssäure enthielt.

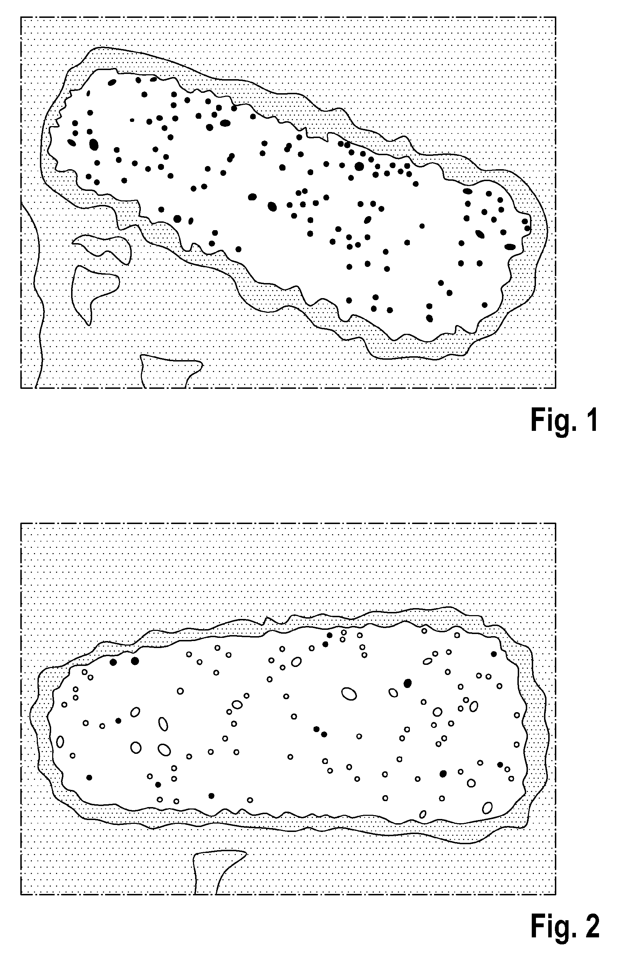

Figur 1 zeigt einen Querschliff durch eine Stentstrebe, die gemäß dem Vergleichsbeispiel oberflächenbehandelt wurde, während Figur 2 eine nach dem erfindungsgemäßen Ausführungsbeispiel 1 behandelte Strebe im Querschliff zeigt. Wie ersichtlich ist die erzeugte Passivierungsschicht wesentlich gleichmäßiger beim erfindungsgemäßen Verfahren, was die weiter oben bereits erläuterten Vorteile bei mechanischer Belastung der Struktur mit sich bringt.

### Ausführungsbeispiel 2 - Anodische Oxidation

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Isopropanol unter Ultraschall gewaschen. Nach mehrfachem Spülen mit deionisiertem Wasser wurde der Stent in nassem Zustand für 5 Minuten in eine auf 30°C erwärmte, wässrige Elektrolytlösung der Zusammensetzung 2.7 g/l NaMnO₄ und 1 g/l NH₄VO₃ getaucht. Weiterhin wurde eine Gleichspannung von 5V zur Verstärkung des Passivierungseffekts angelegt und der Stent als Anode gepolt. Nach dem Herausnehmen des Stents aus der Elektrolytlösung wurde das Implantat mehrfach mit deionisiertem Wasser gespült und danach für 30 Minuten im Umlufttrockner bei 120°C getrocknet.

## Patentansprüche

1. Verfahren zur Herstellung einer korrosionshemmenden Beschichtung auf einem Implantat aus einer biokorrodierbaren Magnesiumlegierung, umfassend die Schritte:
(i) Bereitstellen des Implantats aus einer biokorrodierbaren Magnesiumlegierung; und
(ii) Anodische plasmachemische Behandlung der Implantatsoberfläche in einem wässrigen, fluoridfreien Elektrolyten, der zumindest Ammoniak (NH₃), Phosphorsäure (H₃PO₄) und Borsäure (H₃BO₃) enthält.

2. Verfahren zur Herstellung einer korrosionshemmenden Beschichtung auf einem Implantat aus einer biokorrodierbaren Magnesiumlegierung, umfassend die Schritte:
(i) Bereitstellen des Implantats aus einer biokorrodierbaren Magnesiumlegierung; und
(ii) Anodische Behandlung der Implantatsoberfläche mit einem wässrigen Elektrolyten, der zumindest Natriumpermanganat (NaMnO₄) und Ammoniumvanadat (NH4VO₃) enthält.

3. Implantat mit einer korrosionshemmenden Beschichtung erhalten oder erhältlich nach einem Verfahren gemäß Anspruch 1 oder Anspruch 2.

4. Implantat nach Anspruch 3, bei dem das Implantat ein Stent ist.

5. Implantat nach Anspruch 3 oder 4, bei dem die korrosionshemmende Beschichtung eine Schichtdicke in Bereich von 1 µm bis 10 µm aufweist.

6. Implantat nach einem der Ansprüche 3 bis 5, bei dem die korrosionshemmende Beschichtung zumindest bereichsweise mit einem pharmazeutischen Wirkstoff beladen ist.
